# FASCICULE DE BREVET EUROPEEN

(11) **EP 2 916 807 B1**
(45) Date de publication et mention de la délivrance du brevet: **26.12.2018**
(21) Numéro de dépôt: 13803122.4
(22) Date de dépôt: 08.11.2013
(51) Int. Cl.: A61K 8/365, A61K 8/58, A61Q 5/04, A61K 8/36

(54) **COMPOSITION COMPRENANT UN DERIVE DICARBONYLE ET PROCEDE DE LISSAGE DES CHEVEUX A PARTIR DE CETTE COMPOSITION**
ZUSAMMENSETZUNG MIT EINEM DICARBONYLDERIVAT UND HAARGLÄTTUNGSVERFAHREN UNTER VERWENDUNG DIESER ZUSAMMENSETZUNG
COMPOSITION COMPRISING A DICARBONYL DERIVATIVE AND METHOD FOR STRAIGHTENING THE HAIR USING THIS COMPOSITION

(30) Priorité: 09.11.2012 FR 1260675
(43) Date de publication de la demande: 16.09.2015
(73) Titulaire: L'Oréal, 75008 Paris (FR)
(72) Inventeur: DAUBRESSE, Nicolas, F-78170 La Celle Saint Cloud (FR)
(74) Mandataire: Leray, Noelle
(86) Numéro de dépôt international: PCT/FR2013/052690
(87) Numéro de publication internationale: WO 2014/072657

(56) Documents cités:
- EP-A2- 0 159 628
- WO-A1-2004/012691
- WO-A1-2007/135299
- WO-A1-2011/089985
- WO-A1-2012/080454
- WO-A1-2012/105985
- WO-A2-2011/104282
- WO-A2-2012/010351
- FR-A1- 2 950 531

## Description

La présente invention concerne une composition cosmétique, notamment capillaire, à base d'un ou de plusieurs dérivés dicarbonylés particuliers ainsi qu'un procédé de lissage des fibres kératiniques, notamment des cheveux, à partir de cette composition.

Dans le domaine capillaire, les consommateurs souhaitent disposer de compositions permettant d'apporter un changement temporaire à leur chevelure, et ce en visant une bonne tenue de l'effet réalisé. En général, on désire que le changement persiste aux shampooings pendant au minimum 15 jours, voire plus selon la nature dudit changement. Des traitements existent déjà pour modifier la couleur ou la forme des cheveux ainsi que, dans une certaine mesure, la texture des cheveux. L'un des traitements connus pour modifier la texture des cheveux consiste en l'association de chaleur et d'une composition comprenant du formol. Ce traitement est spécialement efficace pour conférer un meilleur aspect aux cheveux abîmés, et/ou pour traiter les cheveux longs et les cheveux bouclés. On associe l'action du formol à sa capacité de réticuler les protéines par réaction sur ses sites nucléophiles. La chaleur utilisée peut être celle du fer (pince plate ou fer à friser), dont la température peut atteindre en général 200°C ou plus. Toutefois, on cherche de plus en plus à éviter l'emploi de telles substances, qui peuvent se révéler agressives pour le cheveu et les autres matières kératiniques.

Il a ainsi été proposé, par la demande WO2011/104282, un nouveau procédé pour lisser de manière semi-permanente les cheveux, consistant à appliquer une solution d'alphacétoacide sur le cheveu pendant 15 à 120 minutes, puis à sécher et enfin à lisser au fer, à une température d'environ 200°C, la chevelure. L'alphacétoacide employé est de préférence l'acide glyoxylique. Les documents WO2012/010351, WO2012/105985 et WO2007/135299 décrivent également des procédés de lissage des cheveux mettant en jeu des alpha-céto-acides et leurs dérivés.

Toutefois, on a constaté que l'utilisation de l'acide glyoxylique pouvait engendrer quelques limitations importantes; en particulier, à forte concentration il peut ne pas être bien toléré, en particulier quand le cuir chevelu est sensible et/ou irrité. Sa volatilité, amplifiée par l'utilisation de chaleur (fer), peut également poser problème. Par ailleurs, les formulations cosmétiques à pH acide peuvent altérer les cheveux et/ou en altérer la couleur.

Il est déjà connu d'utiliser des esters d'acide glyoxylique dans des compositions capillaires, en particulier dans des compositions de coloration des cheveux tel que décrit dans le document DE19859722 et dans des compositions réductrices tel que décrit dans le document DE19860239.

L'efficacité de ces composés n'est cependant pas encore suffisante.

Le but de l'invention est de développer une composition de lissage/défrisage stable dans le temps et qui permet de lisser/défriser et/ou de réduire le volume les cheveux de façon efficace et rémanente en limitant la dégradation des cheveux tout en conservant un confort au moment de l'application pour l'utilisateur de la composition mais aussi pour le coiffeur qui l'applique.

Ainsi, un objet de la présente invention est une composition cosmétique comprenant
- un ou plusieurs composés dicarbonylés répondant à la formule (I) suivante et/ou leurs hydrates et/ou leurs sels: 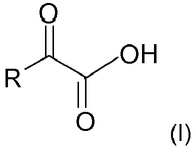 formule (I) dans laquelle
   R représente un atome ou groupe choisi parmi i) hydrogène, ii) carboxy -C(O)OH, iii) alkyle linéaire ou ramifié en C₁-C₆ éventuellement substitué de préférence par au moins un radical hydroxy -OH, carboxy -C(O)-OH ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyl et vii) un radical imidazolylméthyle et ses tautomères tel que 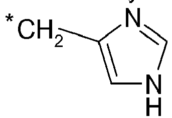 avec * représentant la partie reliée au reste de la molécule,
- un ou plusieurs silanes répondant à la formule (II) suivante et/ou leurs oligomères :

   **R₁Si(OR₂)_{z}(R₃)ₓ(OH)_{y}** **(II)**

   formule (II) dans laquelle :
   - R₁ est une chaîne hydrocarbonée en C₁-C₆, linéaire ou ramifiée, saturée ou insaturée, cyclique ou acyclique substituée par un groupement choisi parmi les groupements :
      - amine NH₂ ou NHR (R= alkyle en C₁-C₂₀, notamment C₁-C₆ éventuellement substitué par un radical comportant un atome de silicium, cycloalkyle en C₃-C₄₀ ou aromatique en C₆-C₃₀),
      - ou par un groupement hydroxy,
      - un groupement thiol,
      - un groupement aryle ou aryloxy substitué ou non, en particulier par un groupement amino ou par un groupement aminoalkyl en C₁-C₄;
      R₁ pouvant être interrompu par un hétéroatome (O, S, NH) ou un groupement carbonyle (CO).
   - R₂ et R₃ identiques ou différents, représentent un groupe alkyle, linéaire ou ramifié, comprenant de 1 à 6 atomes de carbone,
   - Cy désigne un nombre entier allant de 0 à 3, et
   - z désigne un nombre entier allant de 0 à 3, et
   - x désigne un nombre entier allant de 0 à 2,
avec z+x+y=3.

L'invention a aussi pour objet un procédé de lissage des fibres kératiniques, notamment des cheveux, mettant en oeuvre un ou plusieurs composés dicarbonylés répondant à la formule (I) et/ou leurs dérivés et/ou leurs hydrates et/ou leurs sels et un ou plusieurs silanes répondant à la formule (II) et/ou leurs oligomères, le procédé étant tel que défini dans les revendications.

Dans une première variante, le procédé de lissage des cheveux comprend l'application sur les cheveux de la composition de l'invention suivie d'une étape de lissage au moyen d'un fer de lissage à une température d'au moins 150°C, de préférence variant de 150 à 250°C.

Dans une seconde variante, le procédé de lissage des cheveux comprend l'application successive sur les cheveux et dans un ordre quelconque, avec ou sans rinçage intermédiaire, d'une composition comprenant un ou plusieurs composés dicarbonylés répondant à la formule (I) et/ou leurs dérivés et/ou leurs hydrates et/ou leurs sels et d'une composition comprenant un ou plusieurs silanes répondant à la formule (II) et/ou leurs oligomères, l'application de ces compositions étant suivie d'une étape de lissage au moyen d'un fer de lissage à une température d'au moins 150°C, de préférence variant de 150 à 250°C, le procédé étant tel que défini dans les revendications.

L'invention a également pour objet une composition cosmétique susceptible d'être obtenue par mélange d'au moins 2 compositions, l'une comprenant au moins un silane tel que décrit ci-après et l'autre comprenant un ou plusieurs composés dicarbonylés répondant à la formule (I) et/ou leurs dérivés et/ou leurs hydrates et/ou leurs sels tels que décrits ci-après.

La composition de l'invention est stable. La composition de l'invention et le procédé de traitement des fibres kératiniques la mettant en oeuvre permettent un bon lissage des fibres kératiniques en limitant la dégradation de ces fibres kératiniques, même lorsque l'application de la composition est suivie par un traitement thermique, notamment au moyen de fer de lissage des cheveux et présentent une qualité d'usage appréciée, notamment sans vaporisation excessive de la composition au moment du lissage. La composition et le procédé de traitement des fibres kératiniques selon l'invention permettent aussi de limiter le changement de la couleur des fibres, ainsi que les problèmes de casse des fibres telles que les cheveux. La composition et le procédé de l'invention vont en outre améliorer les propriétés physiques des cheveux, en réduisant l'effet de frisottis de façon durable.
Dans ce qui suit l'expression « au moins un » est équivalente à l'expression « un ou plusieurs ».

De préférence, la composition selon l'invention ne comprend ni agent colorant ni agent réducteur.

Par « agents colorants », on entend selon la présente invention des agents de coloration des fibres kératiniques tels que les colorants directs, les pigments ou les précurseurs de colorant d'oxydation (bases et coupleurs). S'ils sont présents, leur teneur ne dépasse pas 0,001 % en poids par rapport au poids total de la composition. En effet, à une telle teneur, seule la composition serait teintée, c'est-à-dire qu'on n'observerait pas d'effet de coloration des fibres kératiniques.

On rappelle que les précurseurs de colorants d'oxydation, bases d'oxydation et coupleurs, sont des composés peu ou non colorés qui par une réaction de condensation en présence d'un agent oxydant, donnent une espèce colorée. Quant aux colorants directs, ces composés sont colorés et présentent une certaine affinité pour les fibres kératiniques.

Par « agent réducteur », on entend selon la présente invention un agent capable de réduire les liaisons disulfures des cheveux, tel que les composés choisis parmi les thiols, les sulfites alcalins, les hydrures, les phosphines.

Dans la présente invention, les composés dicarbonylés de formule (I) ou leurs dérivés peuvent se présenter sous forme libre mais aussi sous leurs formes hydrates ou sous forme de leurs sels, de préférence sous forme libre ou d'hydrates. En tant que « *dérivés* » des composés dicarbonylés de formule (I), on peut citer les esters du ou des groupes carboxy, les amides du ou des groupes carboxy, les (thio)acétals et hémi(thio)acétals de la ou des fonctions carbonyles des composés de formule (I) sous forme libre ou éventuellement sous forme de sels ou d'hydrates, de préférence sous forme libre ou d'hydrates.

Les esters et amides peuvent être synthétisés à partir des procédés d'estérification ou d'amidification classiques à partir des acides correspondants bien connus par l'homme du métier.

Les esters sont par exemple obtenus à partir des acides de formule (I) et d'un mono ou polyalcool.

Par « *mono ou poly alcool* », on entend un composé organique comprenant un groupe hydroxy (monoalcool) ou au moins deux groupes hydroxy (polyalcool ou polyol) ; ledit composé organique hydroxylé pouvant être aliphatique, acyclique, linéaire ou ramifié, ou (hétéro)cyclique, tels que les sucres (mono ou polysaccharides) ou les sucres alcools.

Plus particulièrement, le polyalcool comprend de 2 à 100 groupes hydroxy; et préférentiellement de 2 à 20 groupes hydroxy ; encore plus préférentiellement de 2 à 10 groupes hydroxy, mieux 2 ou 3 groupes hydroxy.

De préférence le mono ou polyalcool est choisi parmi le méthanol, l'éthanol, le propanol, l'isopropanol, le butanol, l'hexanol, l'éthylène glycol, le glycérol, la dihydroxyacétone, le glucose, le sorbitol, le menthol.

Les amides sont par exemple obtenus à partir des acides de formule (I) et d'une mono ou polyamine organique.

Par « mono ou polyamine », on entend un composé organique comprenant un groupe amino(monoamine) ou au moins deux (et de préférence de 2 à 100, mieux de 2 à 20) groupes amino ; le dit composé organique pouvant être aliphatique, acyclique, linéaire ou ramifié ou (hétéro)cyclique.

Par groupe « amino » on entend un groupe amine primaire -NH₂, ou secondaire >NH.

De préférence la mono ou polyamine est aliphatique.

Cette amine est de préférence choisie parmi la méthylamine, l'éthylamine, la propylamine, l'isopropylamine, la butylamine, l'hexylamine, la monoéthanolamine, la monopropanolamine, la propane-1,2,3-triamine et la diaminoacétone.

Les (thio)acétals et hémi(thio)acétals des acides de formule (I) peuvent par exemple être obtenus à partir de la réaction d'alcools pour les acétals ou hémiacétals ou de thiols pour les thioacétals ou hémithioacétals sur des formes bloquées des acides puis hydrolyse. Les alcools peuvent être les mêmes que ceux cités pour les esters. Les thiols peuvent être des équivalents (appelés mono ou polythiols) aux mono ou polyalcools cités supra à ceci près que la ou les fonctions hydroxy desdits mono ou polyalcools sont remplacées par une ou des fonctions thiols SH des mono ou polythiols. Les acétals ou thioacétals peuvent également être des (thio)acétals cycliques.

On peut en particulier citer l'acide diméthoxy acétique, l'acide diéthoxy acétique, l'acide 1,3-dioxane 2 carboxylique, l'acide 1,3-dioxolane 2-carboxylique.

Les sels peuvent être des sels issus de l'interaction des composés de formule (I) avec des acides ou des bases, les acides ou bases pouvant être de nature organique ou minérale.

De préférence les sels sont des sels issus de l'interaction des composés de formule (I) avec des bases. On citera en particuliers les sels de métaux alcalins alcalins ou alcalino terreux et en particulier les sels de sodium.

De préférence, le ou les dérivés dicarbonylés répondant à la formule (I) et/ou leurs dérivés et/ou leurs hydrates et/ou leurs sels sont choisis parmi les dérivés dicarbonylés répondant à la formule (I) dans laquelle R représente i) un atome hydrogène ou ii) un groupe alkyle linéaire ou ramifié en C₁-C₆ éventuellement substitué par un groupe carboxy.

De préférence encore, ils sont choisis parmi l'acide glyoxylique, l'acide pyruvique, l'un de leurs dérivés et leurs hydrates ou leurs sels et encore préférentiellement parmi l'acide glyoxylique, ses dérivés et les formes hydrate de ces composés.

Préférentiellement le ou les composés dicarbonylés de formule (I) de l'invention sont choisis parmi l'acide glyoxylique et ses dérivés et les formes hydrate de ces composés.

On peut citer l'acide glyoxylique ainsi que sa forme hydrate (HO)₂CH-C(O)-OH tel que par exemple l'acide glyoxylique en solution aqueuse à 50 % vendu par la société MERCK.

En tant que dérivés d'acide glyoxylique, on peut citer les esters d'acide glyoxylique, les amides d'acide glyoxylique, les (thio)acétals et hémi(thio)acétals d'acide glyoxylique, les (thio)acétals et hémi(thio)acétals d'ester d'acide glyoxylique.

Les esters d'acide glyoxylique sont par exemple obtenus à partir de l'acide glyoxylique et d'un mono ou polyalcool, notamment ceux cités précédemment.

De préférence le mono ou polyalcool est choisi parmi le méthanol, l'éthanol, le propanol, l'isopropanol, le butanol, l'hexanol, l'éthylène glycol, le glycérol, la dihydroxyacétone, le glucose, le sorbitol, le menthol.

On peut en particulier citer à titre d'esters le glyoxylate de méthyle, le glyoxylate d'éthyle, le glyoxylate de glycérol, le glyoxylate de dihydroxyacétone, le diglyoxylate ou triglyoxylate de glycérol, les mono, di ou tri glyoxylate de sorbitol, les mono, di ou tri glyoxylate de glucose, le glyoxylate de menthyle, leurs acétals, hémiacétals, hydrates.

Les amides d'acide glyoxylique sont par exemple obtenus à partir d'acide glyoxylique et d'une mono ou polyamine organique.

Cette amine est de préférence choisie parmi la méthylamine, l'éthylamine, la propylamine, l'isopropylamine, la butylamine, l'hexylamine, la monoéthanolamine, la monopropanolamine, la propane-1,2,3-triamine et la diaminoacétone.

On peut en particulier citer le Nbétahydroxyéthylamide de l'acide glyoxylique et le Ngammahydroxypropylamide de l'acide glyoxylique leurs (thio)acétals, hémi(thio)acétals, hydrates.

Les (thio)acétals et hémi(thio)acétals d'acide glyoxylique peuvent par exemple être obtenus à partir de la réaction d'alcools ou de thiols pour les thioacétals ou hémithioacétals sur des formes bloquées d'acide glyoxylique puis hydrolyse. Les alcools peuvent être les mêmes que ceux cités pour les esters. Les thiols peuvent être des équivalents (appelés mono ou polythiols) aux mono ou polyalcools cités supra à ceci près que la ou les fonctions hydroxy desdits mono ou polyalcools sont remplacées par une ou des fonctions thiols SH des mono ou polythiols. Les acétals ou thioacétals peuvent également être des (thio)acétals cycliques.

Selon un mode de réalisation particulièrement préféré, le composé de formule (I) est l'acide glyoxylique sous forme hydrate.

Selon un mode de réalisation la composition de l'invention comprend de 0,5 à 15% d'un ou de plusieurs dérivés dicarbonylés répondant à la formule (I) et/ou d'un de leurs dérivés et/ou de leurs formes hydrate et/ou leurs sels, de préférence de 3 à 15%, préférentiellement de 5 à 10 % en poids du poids total de la composition.

Selon l'invention, la composition comprend un ou plusieurs silanes répondant à la formule (II) et/ou leurs oligomères.

Par oligomère on entend les produits de polymérisation des composés de formule (II) comportant de 2 à 10 atomes de silicium.

De préférence, R₂ représente un groupe alkyle comprenant de 1 à 4 atomes de carbone, mieux un groupe alkyle linéaire comprenant de 1 à 4 atomes de carbone, et de préférence le groupe éthyle.

De préférence, R₃ représente un groupe alkyle comprenant de 1 à 4 atomes de carbone, mieux un groupe alkyle linéaire comprenant de 1 à 4 atomes de carbone, et de préférence les groupes méthyle ou éthyle.

De préférence R₁ est une chaîne acyclique.

De préférence le composé de formule (II) ne comporte qu'un atome de silicium dans sa structure.

De préférence, R₁ représente un groupe alkyle, et encore plus préférentiellement un groupe alkyle linéaire, comprenant de 1 à 6 atomes de carbone ou un groupe aminoalkyle en C₁-C₆.

De préférence z varie de 1 à 3. Encore plus préférentiellement z est égal à 3.

De préférence R₁ est une chaîne hydrocarbonée en C1-C6, linéaire ou ramifiée, saturée ou insaturée, substituée par un groupement amine NH2 ou NHR (R= alkyle en C1-C20, notamment C1-C6, cycloalkyle en C3-C40 ou aromatique en C6-C30);

De préférence, la composition comprend au moins un composé de formule (II) choisi parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane, mieux parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane ou leurs oligomères, et de préférence le silane est le 3-aminopropyl triéthoxysilane (APTES) ou ses oligomères.

Le ou les silanes de formule (II) et/ou leurs oligomères peuvent être présents dans la composition selon l'invention en une teneur allant de 0,05 % à 20 %, en particulier de 0,1 à 10 %, de préférence de 0,2 à 5 %, par rapport au poids total de la composition.

De préférence, le ratio pondéral dérivés dicarbonylés répondant à la formule (I) et/ou d'un de leurs dérivés et/ou de leurs formes hydrate et/ou leurs sels/silanes de formule (II) et/ou leurs oligomères varie de 0,1 à 10 mieux de 0,2 à 5.

La composition de l'invention peut comprendre au moins un tensioactif.

Le ou les tensioactifs peuvent être choisi parmi les tensioactifs non ioniques, anioniques, cationiques, amphotères ou zwittérioniques.

Selon un mode de réalisation particulier, la composition comprend au moins un tensioactif amphotère ou zwittérionique.

En particulier, le ou les tensioactifs amphotères ou zwittérioniques, de préférence non siliconés, utilisables dans la présente invention, peuvent être notamment des dérivés d'amines aliphatiques secondaire ou tertiaire, éventuellement quaternisées, dans lesquels le groupe aliphatique est une chaîne linéaire ou ramifiée comportant de 8 à 22 atomes de carbone, lesdits dérivés d'amines contenant au moins un groupe anionique tel que, par exemple, un groupe carboxylate, sulfonate, sulfate, phosphate ou phosphonate.

On peut citer en particulier les alkyl(C₈-C₂₀)bétaïnes, les alkyl(C₈-C₂₀) sulfobétaïnes, les alkyl(C₈-C₂₀)amidoalkyl(C₃-C₈)bétaïnes et les alkyl(C₈-C₂₀)-amidalkyl(C₆-C₈)sulfobétaïnes.

Parmi les dérivés d'amines aliphatiques secondaires ou tertiaires, éventuellement quaternisées utilisables, tels que définis ci-dessus, on peut également citer les composés de structures respectives **(B1)** et **(B2)** suivantes :

Rₐ-C(O)-NH-CH₂-CH₂-N⁺(R_{b})(R_{c})-CH₂C(O)O⁻, M⁺, X⁻ **(B1)**

Formule dans laquelle :
▪ Rₐ représente un groupe alkyle ou alcényle en C₁₀-C₃₀ dérivé d'un acide RₐCOOH, de préférence présent dans l'huile de coprah hydrolysée, un groupe heptyle, nonyle ou undécyle ;
▪ R_{b} représente un groupe bêta-hydroxyéthyle ; et
▪ R_{c} représente un groupe carboxyméthyle ;
▪ M⁺ représente un contre ion cationique issu d'un métal alcalin, alcalinoterreux, tel que le sodium, un ion ammonium ou un ion issu d'une amine organique, et
▪ X⁻ représente un contre ion anionique organique ou inorganique, tel que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate ; ou alors M⁺ et X⁻ sont absents ;

R_{a'}-C(O)-NH-CH₂-CH₂-N(B)(B') **(B2)**

Formule dans laquelle :
▪ B représente le groupe -CH₂-CH₂-O-X' ;
▪ B' représente le groupe -(CH₂)_{z}Y', avec z = 1 ou 2 ;
▪ X' représente le groupe -CH₂-C(O)OH, -CH₂-C(O)OZ', -CH₂-CH₂-C(O)OH, -CH₂-CH₂-C(O)OZ', ou un atome d'hydrogène ;
▪ Y' représente le groupe -C(O)OH, -C(O)OZ', -CH₂-CH(OH)-SO₃H ou le groupe -CH₂-CH(OH)-SO₃-Z' ;
▪ Z' représente un contre ion cationique issu d'un métal alcalin ou alcalinoterreux, tel que le sodium, un ion ammonium ou un ion issu d'une amine organique ;
▪ R_{a'} représente un groupe alkyle ou alcényle en C₁₀-C₃₀ d'un acide R_{a'}-C(O)OH de préférence présent dans l'huile de coprah ou dans l'huile de lin hydrolysée, un groupe alkyle, notamment en C₁₇ et sa forme iso, un groupe en C₁₇ insaturé.

Les composés de ce type sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré

On peut aussi utiliser des composés de formule (B'2) ;

**Ra"-NH-CH(Y")-(CH2)n-C(O)-NH-(CH2)n'-N(Rd)(Re)** **(B'2)**

Formule dans laquelle :
▪ Y" représente le groupe -C(O)OH, -C(O)OZ", -CH2-CH(OH)-SO3H ou le groupe -CH2-CH(OH)-SO3-Z" ;
▪ Rd et Re, indépendamment l'un de l'autre, représentent un radical alkyle ou hydroxyalkyle en C1_C4
▪ Z" représente un contre ion cationique issu d'un métal alcalin ou alcalinoterreux, tel que le sodium, un ion ammonium ou un ion issu d'une amine organique ;
▪ Ra" représente un groupe alkyle ou alcényle en C10-C30 d'un acide Ra"-C(O)OH de préférence présent dans l'huile de coprah ou dans l'huile de lin hydrolysée.
▪ n et n', indépendamment l'un de l'autre, désignent un nombre entier allant de 1 à 3.

Parmi les composés de formule (B'2) on peut citer le composé classé dans le dictionnaire CTFA sous la dénomination sodium diethylaminopropyl cocoaspartamide et commercialisé par la société CHIMEX sous l'appellation CHIMEXANE HB.

Conformément à un mode de réalisation particulier de l'invention, la teneur en tensioactif(s) amphotère(s) ou zwittérionique(s), lorsqu'il(s) est(sont) présent(s), varie de 0,05 à 30 % en poids, de préférence de 0,5 à 10 % en poids, et de manière plus préférée de 0,1 à 5 % en poids, par rapport au poids total de la composition.

La composition selon l'invention peut également comprendre un polymère cellulosique.

Par polymère « *cellulosique* », on entend selon l'invention tout composé polysaccharidique possédant dans sa structure des enchaînements de résidus glucose unis par des liaisons β-1,4 ; outre les celluloses non substituées, les dérivés de celluloses peuvent être anioniques, cationiques, amphotères ou non-ioniques. Ainsi, les polymères cellulosiques de l'invention peuvent être choisis parmi les celluloses non substituées y compris sous une forme microcristalline et les éthers de cellulose. Parmi ces polymères cellulosiques, on distingue les éthers de celluloses, les esters de celluloses et les esters éthers de celluloses. Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulfates d'éthylcellulose.

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.

Ces compositions peuvent être conditionnées dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

Les compositions de l'invention peuvent être aqueuses ou anhydres. Elles sont de préférence aqueuses et comprennent alors de l'eau à une concentration allant de 5 à 98%, mieux de 5 à 90%, encore mieux de 10 à 90 % en poids par rapport au poids total de la composition.

La composition peut notamment comprendre un ou plusieurs solvants organiques notamment hydrosolubles tels que les alcools en C₁-C₇; on peut notamment citer les monoalcools aliphatiques en C₁-C₇ ou aromatiques en C₆-C₇, les polyols et les éthers de polyols en C₃-C₇, qui peuvent être employés seuls ou en mélange avec de l'eau.

La composition de l'invention peut en outre comprendre au moins un ingrédient cosmétique usuel, notamment choisi parmi les propulseurs; les huiles; les corps gras solides et notamment les esters en C₈-C₄₀, les acides en C₈-C₄₀; les alcools en C₈-C₄₀, les filtres solaires; les agents hydratants; les agents antipelliculaires; les agents antioxydants; les agents chélatants; les agents nacrants et opacifiants; les agents plastifiants ou de coalescence; les charges; les silicones et en particulier les polydiméthylsiloxanes; les épaississants ou gélifiants, polymériques ou non autre que les polymères cellulosiques déjà mentionnés; les émulsionnants; les polymères notamment conditionneurs ou coiffants; les parfums; les agents d'alcalinisation tels que la soude ou d'acidification; les silanes ; les agents de réticulation. La composition peut bien évidemment comprendre plusieurs ingrédients cosmétiques figurant dans la liste ci-dessus.

Selon leur nature et la destination de la composition, les ingrédients cosmétiques usuels peuvent être présents en des quantités usuelles, aisément déterminables par l'homme du métier, et qui peuvent être comprises, pour chaque ingrédient, entre 0,01 à 80% en poids. L'homme de métier veillera à choisir les ingrédients entrant dans la composition, ainsi que leurs quantités, de manière à ce qu'ils ne nuisent pas aux propriétés des compositions de la présente invention.

Le pH de la composition est de préférence inférieur à 4, et varie préférentiellement de 1 à 3, mieux de 1,5 à 3, encore mieux de 1,7 à 3.

Il peut être ajusté à la valeur désirée au moyen d'agents alcalinisants et/ou acidifiants habituellement utilisés pour le traitement des fibres kératiniques.

L'agent alcalinisant peut être choisi parmi les agents alcalins minéraux ou organiques ou hybrides ou leurs mélanges.

Le ou les agents alcalins minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.

Le ou les agents alcalins organiques sont de préférence choisis parmi les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

A titre de composés hybrides on peut mentionner les sels des amines citées précédemment avec des acides comme l'acide carbonique, l'acide chlorhydrique.

Le ou les agents alcalins organiques sont par exemple choisis parmi les dérivés aminés tels que alcanolamines, les éthylènediamines oxyéthylénées et/ou oxypropylénées, les acides aminés des amines telles que le 1,3 diaminopropane, le 1,3 diamino 2 propanol, la spermine, la spermidine.

Par alcanolamine on entend une amine organique comprenant une fonction amine primaire, secondaire ou tertiaire, et un ou plusieurs groupements alkyle, linéaires ou ramifiés, en C₁-C₈ porteurs d'un ou plusieurs radicaux hydroxyle.

Convient en particulier à la réalisation de l'invention l'hydroxyde de sodium.

L'agent acidifiant peut être choisi parmi les acides minéraux ou organiques, comme par exemple l'acide chlorhydrique, l'acide phosphorique, l'acide lactique.

La composition selon l'invention se présente de préférence sous forme de gels de coiffage ou de soin, de lotions ou crèmes de soin, de conditionneurs, de masques, de sérums.

La composition selon l'invention peut être obtenue par mélange d'au moins 2 compositions, l'une comprenant au moins un silane tel que décrit ci-après et l'autre comprenant un ou plusieurs composés dicarbonylés répondant à la formule (I) et/ou leurs dérivés et/ou leurs hydrates et/ou leurs sels tels que décrits ci-après.

L'invention a aussi pour objet un procédé de lissage des cheveux mettant en oeuvre un ou plusieurs composés dicarbonylés répondant à la formule (I) et/ou leurs dérivés et/ou leurs hydrates et/ou leurs sels et un ou plusieurs silanes répondant à la formule (II) et/ou leurs oligomères avec une étape de lissage au moyen d'un fer de lissage à une température d'au moins 150°C, de préférence variant de 150 à 250°C

Dans une première variante, le procédé de l'invention comprend l'application de la composition décrite précédemment (composition A) suivi d'une étape de lissage au fer des cheveux de préférence à une température d'au moins 150°C. Le lissage au fer est connu de l'état de la technique. Il consiste à lisser les cheveux avec une pince plate chauffante, généralement métallique. Les fers de lissage sont généralement utilisés à une température variant de 150 à 250°C.

Dans une seconde variante, le procédé de lissage des cheveux comprend l'application successive sur les cheveux et dans un ordre quelconque avec ou sans rinçage intermédiaire d'une composition comprenant un ou plusieurs composés dicarbonylés répondant à la formule (I) et/ou leurs dérivés et/ou leurs hydrates et/ou leurs sels (composition B) et d'une composition comprenant un ou plusieurs silanes répondant à la formule (II) et/ou leurs oligomères (composition C), l'application de ces compositions étant suivie d'une étape de lissage au moyen d'un fer de lissage à une température d'au moins 150°C, de préférence variant de 150 à 250°C.

Dans cette variante on préférera appliquer la composition (C) avant la composition (B).

Le procédé de l'invention peut comprendre d'autres étapes intermédiaires visant à améliorer le lissage des cheveux.

Selon un mode de réalisation particulier, le temps de contact de la composition (A) ou des compositions (B) ou (C) sur les cheveux varie de 10 à 60 minutes, de préférence de 20 à 40 minutes. Après ce ou ces temps de pose, on effectue un lissage à la brosse et au sèche-cheveux (brushing). On lisse ensuite les cheveux au fer à lisser à une température allant de 150 à 250°C, de préférence 210 à 230°C.

Le procédé de l'invention peut comprendre l'application d'autres agents capillaires en pré- ou post traitement. Notamment, il peut comprendre l'application d'un soin de conditionnement en post traitement.

Selon un autre mode de réalisation, le procédé de lissage des cheveux comprend une étape de lavage des cheveux puis de séchage au sèche-cheveux avant application de la composition (A) ou des compositions (B) et (C). Selon ce mode de réalisation particulier, on retrouve ensuite les étapes décrites ci-dessus telles que les temps de contact des compositions, le lissage au fer à lisser, l'application d'un agent de conditionnement et le rinçage, toutes ces étapes pouvant être mise en oeuvre indépendamment l'une de l'autre, un brushing pouvant s'intercaler entre le contact de la composition selon l'invention et le lissage au fer. Selon un mode de réalisation particulier, le lissage au fer à lisser s'effectue en plusieurs passages sur les cheveux, en général 8 à 10 passages.

Le procédé de la présente invention est mis en oeuvre, de préférence, sans une étape de déformation permanente à pH basique ni à base de réducteur.

### Exemples

On réalise les compostions suivantes, les compositions 1 et 2 étant des compositions conformes à l'invention et les compositions C1 à C6 étant des exemples comparatifs.

| | 1 | C1 | C2 | C3 | 2 | C4 |
|---|---|---|---|---|---|---|
| Acide glyoxylique | 5g | 5g | - | - | - | - |
| Acide pyruvique | | - | - | - | 8g | 8g |
| Aminopropyltriéthoxysilane | 5g | - | 5g | 5g | 5g | - |
| Eau | qs 100g | qs 100g | qs 100g | qs 100g | qs 100g | qs 100g |
| Soude/acide chlorhydrique | qs pH 2,2 | qs pH 2,2 | - | qs pH 2,2 | qs pH 2,2 | qs pH 2,2 |

Procédé d'utilisation en un temps :
Les compositions, éventuellement agitées avant emploi, sont appliquées à température ambiante sur des cheveux bouclés qui peuvent être naturels ou colorés, ou sensibilisés par une étape préalable de décoloration à raison de 1g pour 2g de cheveu. Après 15 minutes, les cheveux sont rincés, séchés au sèche-cheveu (brushing) puis lissés par le passage de pinces plates portées à 210°C. Ils sont ultérieurement soumis à des shampooings pour examiner la rémanence des effets de lissage et de modification des propriétés mécaniques et cosmétiques des fibres.

Les compositions C2 et C3 ne permettent pas d'obtenir de lissage permanent. La composition 1 permet d'obtenir des propriétés de lissage supérieures à celles obtenues avec la composition C1 et la composition 2 permet d'obtenir des propriétés de lissage supérieures à celles obtenues avec la composition C4, que ce soit en termes de détente de boucle, de protection de la couleur naturelle comme artificielle, de résistance des fibres aux contraintes mécaniques (traction, frottement, torsion), de brillance, de toucher lisse et de visuel lisse.

Procédé d'utilisation en deux temps :
Suivant un autre procédé en deux étapes sans rinçage, on applique d'une part la composition C2 ou C3, alternativement C1, sur des cheveux, à raison de 1g pour 2g de cheveux, on laisse les compositions agir pendant 15 minutes, puis, sans rincer on applique 1g pour 2g de cheveux de la composition C1, alternativement C2 ou C3 pendant 15 minutes. Les cheveux sont ensuite séchés (brushing) puis lissés par le passage de pinces plates portées à 210°C (10 passages sur mèches séparées en deux épaisseurs).

L'application de la composition C1 associée à l'application des compositions C2 ou C3 conduit à des performances supérieures à celles obtenues avec la composition C1 utilisée seule.

De même, on applique d'une part la composition C2 ou C3, alternativement C4, sur des cheveux, à raison de 1g pour 2g de cheveux, on laisse les compositions agir pendant 15 minutes, puis, sans rincer on applique 1g pour 2g de cheveux de la composition C4, alternativement C2 ou C3 pendant 15 minutes. Les cheveux sont ensuite séchés (brushing) puis lissés par le passage de pinces plates portées à 210°C (10 passages sur mèches séparées en deux épaisseurs).

L'application de la composition C4 associée à l'application des compositions C2 ou C3 conduit à des performances supérieures à celles obtenues avec la composition C4 utilisée seule.

## Revendications

1. Composition cosmétique comprenant
- un ou plusieurs composés dicarbonylés répondant à la formule (I) suivante et/ou leurs hydrates et/ou leurs sels : 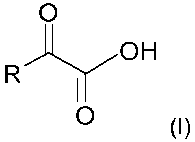 formule (I) dans laquelle
R représente un atome ou groupe choisi parmi i) hydrogène, ii) carboxy -C(O)OH, iii) alkyle linéaire ou ramifié en C₁-C₆ éventuellement substitué de préférence par au moins un radical hydroxy -OH, carboxy -C(O)-OH ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyl et vii) un radical imidazolylméthyle et ses tautomères tel que 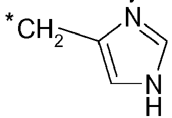 avec * représentant la partie reliée au reste de la molécule,
- un ou plusieurs silanes répondant à la formule (II) suivante et/ou leurs oligomères :
**R₁Si(OR₂)_{z}(R₃)ₓ(OH)_{y}** **(II)**
formule (II) dans laquelle :
• R₁ est une chaîne hydrocarbonée en C₁-C₆, linéaire ou ramifiée, saturée ou insaturée, cyclique ou acyclique substituée par un groupement choisi parmi les groupements :
- amine NH₂ ou NHR (R= alkyle en C₁-C₂₀, notamment C₁-C₆ éventuellement substitué par un radical comportant un atome de silicium, cycloalkyle en C₃-C₄₀ ou aromatique en C₆-C₃₀),
- ou par un groupement hydroxy,
- un groupement thiol,
- un groupement aryle ou aryloxy substitué ou non, en particulier par un groupement amino ou par un groupement aminoalkyl en C₁-C₄;
R₁ pouvant être interrompu par un hétéroatome (O, S, NH) ou un groupement carbonyle (CO).
• R₂ et R₃ identiques ou différents, représentent un groupe alkyle, linéaire ou ramifié, comprenant de 1 à 6 atomes de carbone,
• y désigne un nombre entier allant de 0 à 3, et
• z désigne un nombre entier allant de 0 à 3, et
• x désigne un nombre entier allant de 0 à 2,
avec z+x+y=3.

2. Composition selon la revendication 1, dans laquelle R représente i) un atome d'hydrogène ou ii) un groupe alkyle linéaire ou ramifié en C₁-C₆ éventuellement substitué par un groupe carboxy.

3. Composition selon l'une quelconque des revendications précédentes dans laquelle le ou les composés dicarbonylés répondant à la formule (I) et/ou leurs hydrates et/ou leurs sels sont choisis parmi l'acide glyoxylique, l'acide pyruvique, l'un de leurs sels et leurs hydrates, de préférence parmi l'acide glyoxylique et les formes hydrate de ces composés.

4. Composition selon la revendication 3, dans laquelle l'acide glyoxylique se présente sous sa forme hydrate.

5. Composition selon l'une quelconque des revendications 1 à 4, comprenant de 0,5 à 15% en poids d'un ou de plusieurs dérivés dicarbonylés répondant à la formule (I) et/ou leurs hydrates et/ou leurs sels, de préférence de 3 à 15%, préférentiellement de 5 à 10 % en poids du poids total de la composition.

6. Composition selon l'une quelconque des revendications 1 à 5, dans laquelle R₂ représente un groupe alkyle comprenant de 1 à 4 atomes de carbone, mieux un groupe alkyle linéaire comprenant de 1 à 4 atomes de carbone, et de préférence le groupe éthyle et R₃ représentent un groupe alkyle comprenant de 1 à 4 atomes de carbone, mieux un groupe alkyle linéaire comprenant de 1 à 4 atomes de carbone, et de préférence les groupes méthyle ou éthyle.

7. Composition selon l'une quelconque des revendications précédentes, **caractérisée en ce que** R₁ représente un groupe aminoalkyle en C₁-C₆.

8. Composition selon selon l'une quelconque des revendications précédentes, **caractérisée en ce que** z varie de 1 à 3, de préférence z est égal à 3.

9. Composition selon l'une quelconque des revendications précédentes, **caractérisée en ce que** R₁ est une chaîne acyclique et que le composé de formule (II) ne comporte qu'un atome de silicium dans sa structure.

10. Composition selon l'une quelconque des revendications précédentes, **caractérisée en ce qu'**elle comprend au moins un composé de formule (II) choisi parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane, mieux parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane ou leurs oligomères.

11. Composition selon l'une quelconque des revendications précédentes, **caractérisée en ce qu'**elle comprend au moins un composé choisi parmi le 3-aminopropyl triéthoxysilane et ses oligomères.

12. Composition selon l'une des revendications précédentes, **caractérisée en ce que** le ou les silanes de formule (II) et/ou leurs oligomères sont présents en une teneur en matière active allant de 0,05 à 20% en poids, de préférence de 0,1 à 10 en poids, de préférence allant de 0,2 à 5 % en poids par rapport au poids total de la composition.

13. Composition selon l'une quelconque des revendications précédentes dans laquelle le ratio pondéral dérivés dicarbonylés répondant à la formule (I) et/ou de leurs formes hydrate et/ou leurs sels/silanes de formule (II) et/ou leurs oligomères varie de 0,1 à 10 mieux de 0,2 à 5.

14. Composition selon l'une quelconque des revendications précédentes **caractérisée en ce qu'**elle est aqueuse et comprend de l'eau à une concentration de préférence allant de 5 à 98%, mieux de 5 à 90%, encore mieux de 10 à 90 % en poids par rapport au poids total de la composition.

15. Composition selon l'une quelconque des revendications précédentes **caractérisée en ce qu'**elle présente un pH inférieur à 4, le pH variant préférentiellement de 1 à 3, mieux de 1,5 à 3, encore mieux de 1,7 à 3.

16. Composition selon l'une quelconque des revendications précédentes **caractérisée par le fait qu'**elle est susceptible d'être obtenue par mélange de deux compositions, l'une comprenant au moins un silane tel que décrit à l'une des revendications 1 et 6 à 12 et l'autre comprenant un ou plusieurs composés dicarbonylés répondant à la formule (I) et/ou leurs hydrates et/ou leurs sels tels que décrits à l'une quelconque des revendications 1 à 5.

17. Procédé de lissage des fibres kératiniques, notamment des cheveux mettant en oeuvre un ou plusieurs composés dicarbonylés répondant à la formule (I) et/ou leurs dérivés choisis parmi les esters du ou des groupes carboxy, les amides du ou des groupes carboxy, les (thio)acétals et hémi(thio)acétals de la ou des fonctions carbonyles des composés de formule (I) sous forme libre ou éventuellement sous forme de sels ou d'hydrates, et/ou leurs hydrates et/ou leurs sels tels que décrits à l'une quelconque des revendications 1 à 5 et un ou plusieurs silanes répondant à la formule (II) et/ou leurs oligomères tels que décrits à l'une des revendications 1 et 6 à 12 avec une étape de lissage au moyen d'un fer de lissage à une température d'au moins 150°C, de préférence allant de 150 à 250°C:

18. Procédé de lissage selon la revendication 17 qui comprend l'application sur les cheveux de la composition selon l'une quelconque des revendications 1 à 16, suivie d'un temps de contact compris entre 10 et 60 minutes suivi d'une étape de lissage au moyen d'un fer de lissage à une température d'au moins 150°C, de préférence entre 150 et 250°C:

19. Procédé de lissage selon la revendication 17 qui comprend l'application successive sur les cheveux et dans un ordre quelconque, avec ou sans rinçage intermédiaire, d'une composition comprenant un ou plusieurs composés dicarbonylés répondant à la formule (I) et/ou leurs dérivés esters du ou des groupes carboxy, les amides du ou des groupes carboxy, les (thio)acétals et hémi(thio)acétals de la ou des fonctions carbonyles des composés de formule (I) sous forme libre ou éventuellement sous forme de sels ou d'hydrates, et/ou leurs hydrates et/ou leurs sels (composition B) et d'une composition comprenant un ou plusieurs silanes répondant à la formule (II) et/ou leurs oligomères (composition C), l'application de ces compositions étant suivie d'un temps de contact pour chacune d'entre elles allant de 10 à 60 minutes puis d'une étape de lissage au moyen d'un fer de lissage à une température d'au moins 150°C, de préférence allant de 150 à 250°C.

20. Utilisation de la composition selon l'une quelconque des revendications 1 à 16 pour le lissage/défrisage des fibres kératiniques, notamment des cheveux.

## Patentansprüche

1. Kosmetische Zusammensetzung, umfassend
- eine oder mehrere Dicarbonylverbindungen gemäß nachstehender Formel (I) und/oder Hydrate davon und/oder Salze davon: 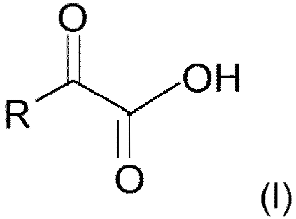 wobei in Formel (I)
R für ein Atom oder eine Gruppe steht, das bzw. die ausgewählt ist aus: i) Wasserstoff, ii) Carboxy -C(O)OH, iii) geradkettigem oder verzweigtem C₁-C₆-Alkyl, das gegebenenfalls substituiert ist, vorzugsweise durch mindestens einen Hydroxylrest -OH, Carboxylrest -C(O)OH oder Halogenrest wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie 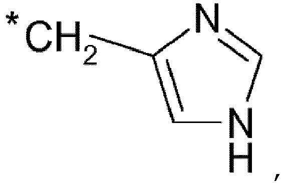 wobei * für den mit dem Rest des Moleküls verknüpften Teil steht,
- ein oder mehrere Silane der nachstehenden Formel (II) und/oder Oligomere davon:
**R₁Si(OR₂)_{z}(R₃)ₓ(OH)_{y}** **(II)**
wobei in der Formel (II):
• R₁ für eine lineare oder verzweigte, gesättigte oder ungesättigte, cyclische oder acyclische C₁-C₆-Kohlenwasserstoffkette, die durch eine Gruppe, die ausgewählt ist aus den Gruppen:
- Amin NH₂ oder NHR (R = C₁-C₂₀-Alkyl, insbesondere C₁-C₆-Alkyl, das gegebenenfalls durch einen ein Siliciumatom enthaltenden Rest, einen C₃-C₄₀-Cycloalkylrest oder einen aromatischen C₆-C₃₀-Rest substituiert ist),
- oder durch eine Hydroxygruppe,
- eine Thiolgruppe,
- eine Aryl- oder Aryloxygruppe, die gegebenenfalls substituiert ist, insbesondere durch eine Aminogruppe oder durch eine C₁-C₄-Aminoalkylgruppe,
substituiert ist, steht, wobei R₁ durch ein Heteroatom (O, S, NH) oder eine Carbonylgruppe (CO) unterbrochen sein kann,
• R₂ und R₃ gleich oder verschieden sind und für eine lineare oder verzweigte Alkylgruppe mit 1 bis 6 Kohlenstoffatomen stehen,
• y für eine ganze Zahl im Bereich von 0 bis 3 steht und
• z für eine ganze Zahl im Bereich von 0 bis 3 steht und
• x für eine ganze Zahl im Bereich von 0 bis 2 steht,
mit z + x + y = 3.

2. Zusammensetzung nach Anspruch 1, wobei R für i) ein Wasserstoffatom oder ii) eine geradkettige oder verzweigte C₁-C₆-Alkylgruppe, die gegebenenfalls durch eine Carboxygruppe substituiert ist, steht.

3. Zusammensetzung nach einem der vorhergehenden Ansprüche, wobei die Dicarbonylverbindung(en) der Formel (I) und/oder Hydrate davon und/oder Salze davon aus Glyoxylsäure, Brenztraubensäure, einem ihrer Salze und ihrer Hydrate, vorzugsweise aus Glyoxylsäure und den Hydratformen dieser Verbindungen, ausgewählt ist bzw. sind.

4. Zusammensetzung nach Anspruch 3, wobei die Glyoxylsäure in ihrer Hydratform vorliegt.

5. Zusammensetzung nach einem der Ansprüche 1 bis 4, umfassend 0,5 bis 15 Gew.-% eines oder mehrerer Dicarbonylderivate der Formel (I) und/oder Hydrate davon und/oder Salze davon, vorzugsweise 3 bis 15 Gew.-%, bevorzugt 5 bis 10 Gew.-%, bezogen auf das Gesamtgewicht der Zusammensetzung.

6. Zusammensetzung nach einem der Ansprüche 1 bis 5, wobei R₂ für eine Alkylgruppe mit 1 bis 4 Kohlenstoffatomen, besser eine lineare Alkylgruppe mit 1 bis 4 Kohlenstoffatomen und vorzugsweise die Ethylgruppe steht und R₃ für eine Alkylgruppe mit 1 bis 4 Kohlenstoffatomen, besser eine lineare Alkylgruppe mit 1 bis 4 Kohlenstoffatomen und vorzugsweise Methyl- oder Ethylgruppen steht.

7. Zusammensetzung nach einem der vorhergehenden Ansprüche, **dadurch gekennzeichnet, dass** R₁ für eine C₁-C₆-Aminoalkylgruppe steht.

8. Zusammensetzung nach einem der vorhergehenden Ansprüche, **dadurch gekennzeichnet, dass** z im Bereich von 1 bis 3 liegt und vorzugsweise z = 3 ist.

9. Zusammensetzung nach einem der vorhergehenden Ansprüche, **dadurch gekennzeichnet, dass** R₁ für eine acyclische Kette steht und dass die Verbindung der Formel (II) kein Siliciumatom in ihrer Struktur enthält.

10. Zusammensetzung nach einem der vorhergehenden Ansprüche, **dadurch gekennzeichnet, dass** sie eine Verbindung der Formel (II), die aus 3-Aminopropyltriethoxysilan (APTES), 3-Aminoethyltriethoxysilan (AETES), 3-Aminopropylmethyldiethoxysilan, N-(2-Aminoethyl)-3-aminopropyltriethoxysilan, 3-(m-Aminophenoxy)propyltrimethoxysilan, p-Aminophenyltrimethoxysilan und N-(2-Aminoethylaminomethyl)phenethyltrimethoxysilan, besser aus 3-Aminopropyltriethoxysilan (APTES), 3-Aminoethyltriethoxysilan (AETES), 3-Aminopropylmethyldiethoxysilan und N-(2-Aminoethyl)-3-aminopropyltriethoxysilan, oder Oligomeren davon ausgewählt ist, umfasst.

11. Zusammensetzung nach einem der vorhergehenden Ansprüche, **dadurch gekennzeichnet, dass** sie mindestens eine Verbindung, die aus 3-Aminopropyltriethoxysilan und Oligomeren davon ausgewählt ist, umfasst.

12. Zusammensetzung nach einem der vorhergehenden Ansprüche, **dadurch gekennzeichnet, dass** das Silan bzw. die Silane der Formel (II) und/oder Oligomere davon in einem Aktivmaterialgehalt im Bereich von 0,05 bis 20 Gew.-%, vorzugsweise 0,1 bis 10 Gew.-%, vorzugsweise im Bereich von 0,2 bis 5 Gew.-%, bezogen auf das Gesamtgewicht der Zusammensetzung, vorliegt bzw. vorliegen.

13. Zusammensetzung nach einem der vorhergehenden Ansprüche, wobei das Gewichtsverhältnis von Dicarbonylderivaten der Formel (I) und/oder Hydratformen davon und/oder Salzen davon zu Silanen der Formel (II) und/oder Oligomeren davon im Bereich von 0,1 bis 10 und besser von 0,2 bis 5 liegt.

14. Zusammensetzung nach einem der vorhergehenden Ansprüche, **dadurch gekennzeichnet, dass** sie wässrig ist und Wasser in einer Konzentration, die vorzugsweise im Bereich von 5 bis 98 Gew.-%, besser von 5 bis 90 Gew.-%, noch besser von 10 bis 90 Gew.-%, bezogen auf das Gesamtgewicht der Zusammensetzung, liegt, umfasst.

15. Zusammensetzung nach einem der vorhergehenden Ansprüche, **dadurch gekennzeichnet, dass** sie einen pH-Wert von weniger als 4 aufweist, wobei der pH-Wert vorzugsweise im Bereich von 1 bis 3, besser von 1,5 bis 3, noch besser von 1,7 bis 3, variiert.

16. Zusammensetzung nach einem der vorhergehenden Ansprüche, **dadurch gekennzeichnet, dass** sie durch Mischen von zwei Zusammensetzungen erhältlich ist, wobei die eine mindestens ein Silan gemäß einem der Ansprüche 1 und 6 bis 12 umfasst und die andere eine oder mehrere Dicarbonylverbindungen der Formel (I) und/oder Hydrate davon und/oder Salze davon gemäß einem der Ansprüche 1 bis 5 umfasst.

17. Verfahren zum Glätten von Keratinfasern, insbesondere dem Haar unter Verwendung einer oder mehrerer Dicarbonylverbindungen der Formel (I) und/oder Derivaten davon, die aus Estern der Carboxylgruppe(n), Amiden der Carboxylgruppe(n), (Thio)-acetalen und Hemi(thio)acetalen der Carbonylfunktion(en) der Verbindungen der Formel (I) in freier Form oder gegebenenfalls in Form von Salzen oder Hydraten ausgewählt sind, und/oder Hydraten und/oder Salzen davon gemäß einem der Ansprüche 1 bis 5 und eines oder mehrerer Silane der Formel (II) und/oder Oligomeren davon gemäß einem der Ansprüche 1 und 6 bis 12 mit einem Glättungsschritt unter Verwendung eines Glätteisens bei einer Temperatur von mindestens 150 °C, vorzugsweise im Bereich von 150 bis 250 °C.

18. Glättungsverfahren nach Anspruch 17, bei dem man auf das Haar die Zusammensetzung nach einem der Ansprüche 1 bis 16 aufbringt, worauf eine Kontaktzeit zwischen 10 und 60 Minuten folgt, worauf ein Glättungsschritt unter Verwendung eines Glätteisens bei einer Temperatur von mindestens 150 °C, vorzugsweise zwischen 150 und 250 °C, folgt.

19. Glättungsverfahren nach Anspruch 17, bei dem man auf das Haar aufeinanderfolgend und in beliebiger Reihenfolge mit oder ohne Zwischenspülung eine Zusammensetzung, die eine oder mehrere Dicarbonylverbindungen der Formel (I) und/oder Esterderivate der Carboxylgruppe(n), Amide der Carboxylgruppe(n), (Thio)acetale und Hemi(thio)acetale der Carbonylfunktion(en) der Verbindungen der Formel (I) in freier Form oder gegebenenfalls in Form von Salzen oder Hydraten, und/oder Hydrate davon und/oder Salze davon umfasst (Zusammensetzung B), und eine Zusammensetzung, die ein oder mehrere Silane der Formel (II) und/oder Oligomere davon umfasst (Zusammensetzung C), aufbringt, wobei auf das Aufbringen dieser Zusammensetzungen eine Kontaktzeit für jede davon im Bereich von 10 bis 60 Minuten und dann ein Glättungsschritt unter Verwendung eines Glätteisens bei einer Temperatur von mindestens 150 °C, vorzugsweise im Bereich von 150 bis 250 °C, folgt.

20. Verwendung der Zusammensetzung nach einem der Ansprüche 1 bis 16 zum Glätten/Entkräuseln von Keratinfasern, insbesondere dem Haar.

## Claims

1. Cosmetic composition comprising:
- one or more dicarbonyl compounds corresponding to formula (I) below, and/or hydrates thereof and/or salts thereof: 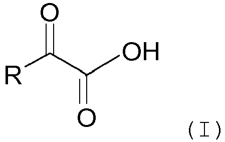 in which formula (I):
R represents an atom or group chosen from i) hydrogen, ii) carboxy -C(O)-OH, iii) linear or branched C₁-C₆ alkyl which is optionally substituted, preferably with at least one hydroxyl -OH radical, carboxy -C(O)-OH radical or halogen such as Br; iv) optionally substituted phenyl, v) optionally substituted benzyl, iv) and v) preferably being optionally substituted with at least one -OH or -C(O)-OH radical; vi) an indolyl radical and vii) an imidazolylmethyl radical and tautomers thereof such as 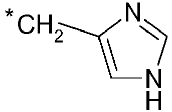 with * representing the part linked to the rest of the molecule,
- one or more silanes corresponding to formula (II) below and/or oligomers thereof:
**R₁Si(OR₂)_{z}(R₃)ₓ(OH)_{y}** **(II)**
in which formula (II):
• R₁ is a linear or branched, saturated or unsaturated, cyclic or acyclic C₁-C₆ hydrocarbon-based chain substituted with a group chosen from the following groups:
- amine NH₂ or NHR (R = C₁-C₂₀ and especially C₁-C₆ alkyl optionally substituted with a radical comprising a silicon atom, C₃-C₄₀ cycloalkyl or C₆-C₃₀ aromatic),
- or with a hydroxyl group,
- a thiol group,
- an aryl or aryloxy group which is unsubstituted or substituted, in particular with an amino group or with a C₁-C₄ aminoalkyl group;
R₁ possibly being interrupted with a heteroatom (O, S or NH) or a carbonyl group (CO),
• R₂ and R₃, which may be identical or different, represent a linear or branched alkyl group comprising from 1 to 6 carbon atoms,
• y denotes an integer ranging from 0 to 3, and
• z denotes an integer ranging from 0 to 3, and
• x denotes an integer ranging from 0 to 2,
with z + x + y = 3.

2. Composition according to Claim 1, in which R represents i) a hydrogen atom or ii) a linear or branched C₁-C₆ alkyl group optionally substituted with a carboxyl group.

3. Composition according to either of the preceding claims, in which the dicarbonyl compound(s) corresponding to formula (I) and/or hydrates thereof and/or salts thereof are chosen from glyoxylic acid and pyruvic acid, one of the salts thereof and hydrates thereof, preferably from glyoxylic acid the hydrate forms of these compounds.

4. Composition according to Claim 3, in which the glyoxylic acid is in its hydrate form.

5. Composition according to any one of Claims 1 to 4, comprising from 0.5% to 15% by weight of one or more dicarbonyl derivatives corresponding to formula (I) and/or hydrates thereof and/or salts thereof, preferably from 3% to 15% and preferentially from 5% to 10% by weight relative to the total weight of the composition.

6. Composition according to any one of Claims 1 to 5, in which R₂ represents an alkyl group comprising from 1 to 4 carbon atoms, better still a linear alkyl group comprising from 1 to 4 carbon atoms, and preferably the ethyl group, and R₃ represents an alkyl group comprising from 1 to 4 carbon atoms, better still a linear alkyl group comprising from 1 to 4 carbon atoms, and preferably methyl or ethyl groups.

7. Composition according to any one of the preceding claims, **characterized in that** R₁ represents a C₁-C₆ aminoalkyl group.

8. Composition according to any one of the preceding claims, **characterized in that** z ranges from 1 to 3 and preferably z is equal to 3.

9. Composition according to any one of the preceding claims, **characterized in that** R₁ is an acyclic chain and **in that** the compound of formula (II) comprises only one silicon atom in its structure.

10. Composition according to any one of the preceding claims, **characterized in that** it comprises at least one compound of formula (II) chosen from 3-aminopropyltriethoxysilane (APTES), 3-aminoethyltriethoxysilane (AETES), 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane and N-(2-aminoethylaminomethyl)phenethyltrimethoxysilane, better still from 3-aminopropyltriethoxysilane (APTES), 3-aminoethyltriethoxysilane (AETES), 3-aminopropylmethyldiethoxysilane and N-(2-aminoethyl)-3-aminopropyltriethoxysilane or oligomers thereof.

11. Composition according to any one of the preceding claims, **characterized in that** it comprises at least one compound chosen from 3-aminopropyltriethoxysilane and oligomers thereof.

12. Composition according to one of the preceding claims, **characterized in that** the silane(s) of formula (II) and/or the oligomers thereof are present in an active material content ranging from 0.05% to 20% by weight, preferably from 0.1% to 10% by weight, preferably ranging from 0.2% to 20% by weight, relative to the total weight of the composition.

13. Composition according to any one of the preceding claims, in which the weight ratio dicarbonyl derivatives corresponding to formula (I) and/or of hydrate forms thereof and/or salts/silanes thereof of formula (II) and/or oligomers thereof ranges from 0.1 to 10 and better still from 0.2 to 5.

14. Composition according to any one of the preceding claims, **characterized in that** it is aqueous and comprises water in a concentration preferably ranging from 5% to 98%, better still from 5% to 90% and even better still from 10% to 90% by weight relative to the total weight of the composition.

15. Composition according to any one of the preceding claims, **characterized in that** it has a pH of less than 4, the pH preferentially ranging from 1 to 3, better still from 1.5 to 3 and even better still from 1.7 to 3.

16. Composition according to any one of the preceding claims, **characterized in that** it may be obtained by mixing two compositions, one comprising at least one silane as described in one of Claims 1 and 6 to 12 and the other comprising one or more dicarbonyl compounds corresponding to formula (I) and/or hydrates thereof and/or salts thereof as described in any one of Claims 1 to 5.

17. Process for straightening keratin fibers, especially the hair, using one or more dicarbonyl compounds corresponding to formula (I) and/or derivatives thereof chosen from esters of the carboxyl group(s), amides of the carboxyl group(s), (thio)acetals and hemi(thio)acetals of the carbonyl function (s) of the compounds of formula (I) in free form or optionally in the form of salts or hydrates, and/or hydrates thereof and/or salts thereof as described in any one of Claims 1 to 5 and one or more silanes corresponding to formula (II) and/or oligomers thereof as described in one of Claims 1 and 6 to 12, with a straightening step using a straightening iron at a temperature of at least 150°C, preferably ranging from 150 to 250°C.

18. Straightening process according to Claim 17, which comprises the application to the hair of the composition according to any one of Claims 1 to 16, followed by a contact time of between 10 and 60 minutes, followed by a straightening step using a straightening iron at a temperature of at least 150°C, preferably between 150 and 250°C.

19. Straightening process according to Claim 17, which comprises the successive application to the hair, in any order, with or without intermediate rinsing, of a composition comprising one or more dicarbonyl compounds corresponding to formula (I) and/or ester derivatives of the carboxyl group(s), amides of the carboxyl group(s), (thio)acetals and hemi(thio)acetals of the carbonyl function(s) of the compounds of formula (I) in free form or optionally in the form of salts or hydrates, and/or hydrates thereof and/or salts thereof (composition B) and a composition comprising one or more silanes corresponding to formula (II) and/or oligomers thereof (composition C), the application of these compositions being followed by a time of contact for each of them ranging from 10 to 60 minutes and then a straightening step using a straightening iron at a temperature of at least 150°C, preferably ranging from 150 to 250°C.

20. Use of the composition according to any one of Claims 1 to 16, for straightening/relaxing keratin fibers, especially the hair.
